# EUROPEAN PATENT APPLICATION

(11) **EP 2 374 414 A1**
(43) Date of publication of application: **12.10.2011**
(21) Application number: 10159492.7
(22) Date of filing: 09.04.2010
(51) Int. Cl.: A61B 10/00, C12M 1/20, B65D 51/14, B65D 53/02

(54) **Biopsy dish and closure element therefor**

(30) Priority: 07.04.2010 US 755879
(71) Applicant: 3088081 Canada Inc., St-Hilaire, Québec J3G 4S5 (CA)
(72) Inventor: Lafond, André, St-Hilaire Québec J3G 4S5 (CA); Leblanc, Daniel, Vercheres Québec J0L 2R0 (CA)
(74) Representative: Texier, Christian

(57) **Abstract**

A sampling dish (10), comprising a body (12) defining a cavity (22) and comprising a bottom wall (16), an open top end (20) and side walls (18) extending from the bottom wall to the open top end; and a removable closure element (14) comprising a ring (34) and a plate, the ring being movable into a mating position with edges of the side walls of the body to secure securing said plate in place over the open top of the body, thereby ensuring hermeticity of the dish in a closed position, and the ring being removable from the mating position to allow removal of the plate from the open top of the body for access to the cavity of the dish in an open position.

## Description

### FIELD OF THE INVENTION

The present invention relates to biopsy dishes. More specifically, the present invention is concerned with a sampling dish and a closure element therefor.

### BACKGROUND OF THE INVENTION

For taking a biopsy of an organ, samples are typically taken from a plurality of locations in the organ to increase the likelihood of locating a tumor for example, in the case of cancer prevention. For example, in the case of biopsy of the prostate, usually between about 8 and 12 tissue samples, of a size of about 1-1.5 mm by 20-25 mm or 10-15 mm, are collected from different locations.

Typically, the collection location and the processing laboratory are separated spatially and also in time, as, generally, analysis of the biological samples at the time of collection is either impossible or not practical. Therefore, the samples need to be identified with information about the site where they were collected, and stored with a suitable preservative, such as for example formalin, and then sent to a remote location for analysis.

US 6,258,327 patent to Tatum teaches a device for collecting, storing and transporting medical biopsy specimen. The device comprises a tray supporting individual separate preservative-filled chambers mounted over a schematic diagram of the anatomical organ or organ system from which the specimens are being collected, and an identification panel having a key for each chamber.

US 7,147,826 to Haywood et al. teaches a device for receiving a biological sample, comprising a container with a cover, and a sample holder removably received in the container.

US 2007/0183938 application publication, to Booker discloses a device comprising one more tissue cassettes, each receiving a tissue sample from a different region of the organ of interest and each provided with an indentifying indicia.

US 2008/0113425 application publication to Cortelazzo teaches a container containing stacked sample containment cups and a distributor allowing distribution of a preservative liquid inside the cups.

US 2009/0183581 application publication to Wilkinson describes a container comprising separate chambers, each chamber being able to receive and accommodate a sample holder.

There is still a need in the art for a simple, easy to use and hermetic sampling dish.

### SUMMARY OF THE INVENTION

More specifically, there is provided a sampling dish, comprising a body defining a cavity, said body comprising a bottom wall, an open top end and side walls extending from said bottom wall to said open top end; a plate, and a ring, said ring being movable into a mating position with edges of the side walls of said body to secure said plate in place over the open top of the body, thereby ensuring hermeticity of the dish in a closed position, and said ring being removable from said mating position to allow removal of said plate from said open top of the body for access to said cavity of the dish in an open position.

Other objects, advantages and features of the present invention will become more apparent upon reading of the following non-restrictive description of embodiments thereof, given by way of example only with reference to the accompanying drawings.

### BRIEF DESCRIPTION OF THE DRAWINGS

In the appended drawings:

Figure 1 is a perspective view of a sampling dish according to an embodiment of a first aspect of the present invention;

Figure 2 is a perspective view of a sampling dish according to another embodiment of the first of the present invention;

Figure 3 is a perspective view of a sampling dish according to another embodiment of the first aspect of the present invention;

Figure 4 is a perspective view of a closure element according to an embodiment of a second aspect of the present invention;

Figure 5 is a cut off view of the closure element of Figure 4;

Figure 6 is an exploded view of a detail of Figure 5;

Figure 7 is a detail of Figure 5;

Figure 8 shows the closure element of Figure 5 positioned on top of a base;

Figure 9 is a top view from a sampling dish according to an embodiment of the present invention; and

Figure 10 shows a detail of a ring of the closure element of Figure 5.

### DESCRIPTION OF EMBODIMENTS OF THE INVENTION

A sampling dish 10 according to an embodiment of an aspect of the present invention generally comprises a body 12 defining a cavity 22, and a removable closure element 14.

The body 12 as illustrated has a general cylindrical shape, with a bottom wall 16 and a side wall 18 extending from the bottom wall 16 to an open top end 20, and defining the inner cavity 22.

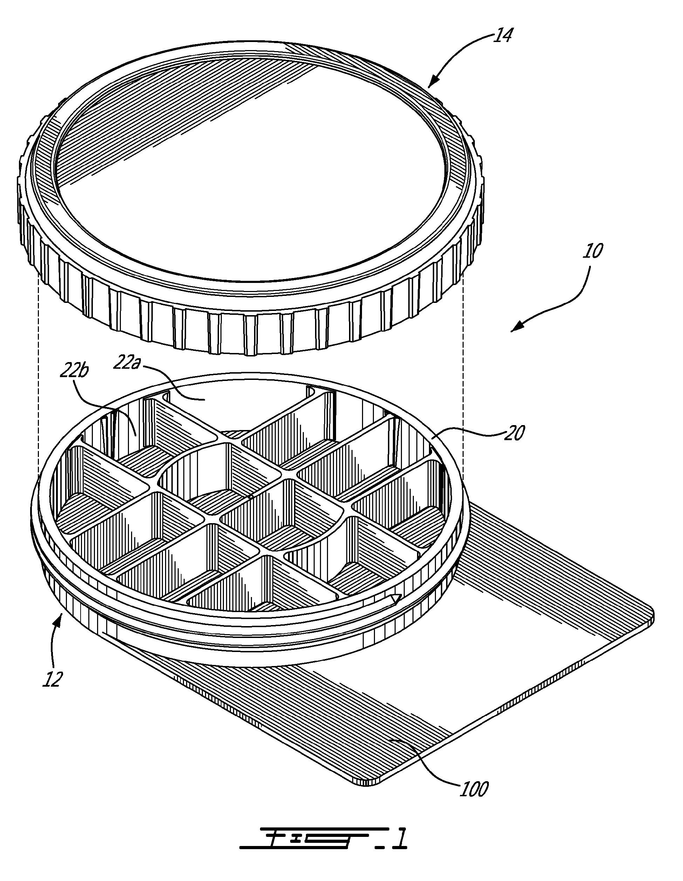

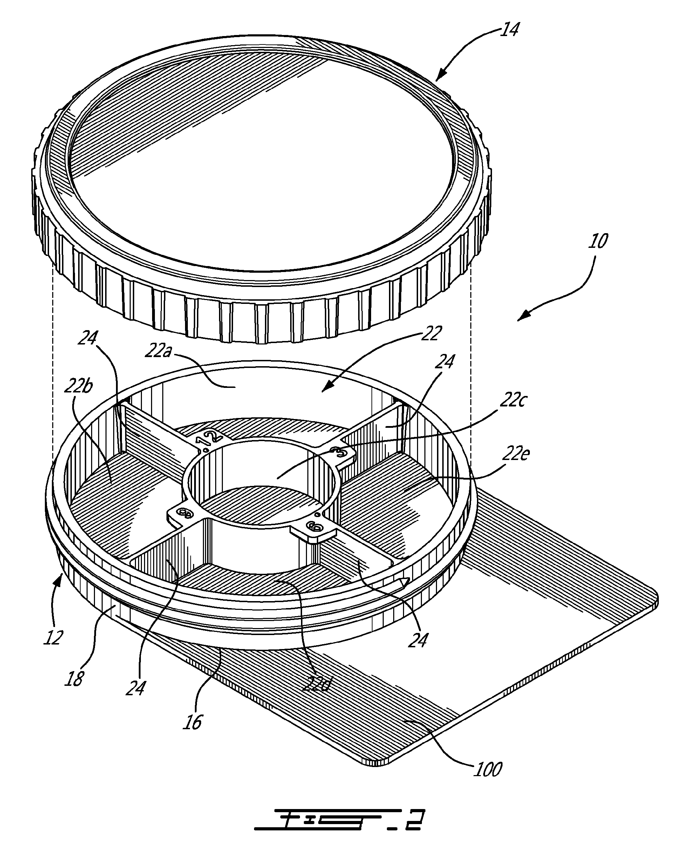

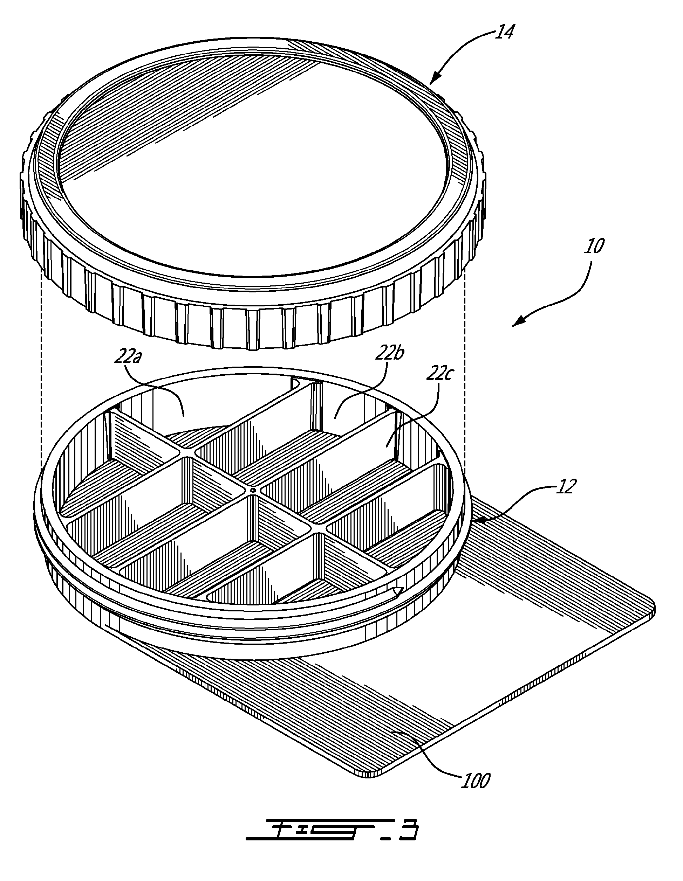

The cavity 22 is divided into separate adjacent compartments 22a, 22b, 22c... Each compartment may be pre-labelled with a designation of the site from which the corresponding tissue sample is to be collected, or with a number as shown in Figure 1. As shown in Figures 1 to 3, each compartment can be of any shape, such as round, square, rectangular for example, and of a variable size within the cavity 22, adapted to accommodate the anticipated tissue sample from a particular site and an adequate amount of preservative.

Typically the side wall 16 of the body 12, i.e. the depth of the cavity 22, is relatively short.

Thus, in one single container of fixative, biopsy samples from various locations within a particular anatomical organ or system are placed in adjacent, separate and individual preservative-filled compartments of a single dish, each compartment being pre-labelled with a specific biopsy site. The label can be etched or printed on the floor of each compartment for example, on an upper edge of the walls 24 as shown for example in Figure 1. Alternatively, the label may be positioned, face-up, on the exterior surface of the bottom face of the base, so as to be read through the transparent material of the base. This makes it easy for medical staff to use. As shown in Figures 1-3, 8 and 9, the body 12 may be further provided with an extension plate 100, for support of a patient identification label for example.

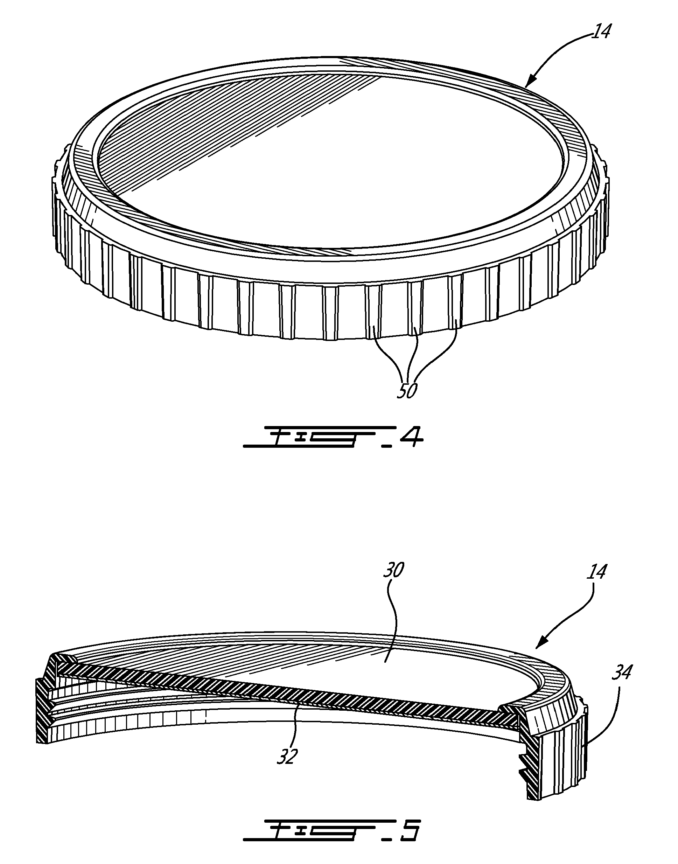

As best seen in Figures 4-6, the closure member 14 according to an embodiment of an aspect of the present invention comprises a ring 34 and a plate formed of an inner lid 30 and a gasket 32.

The ring 34 is of a diameter slightly larger than an exterior upper diameter of the body 12. The ring 34 comprises a side wall 36 of a generally cylindrical shape with an outer surface 38 and an inner surface 40.

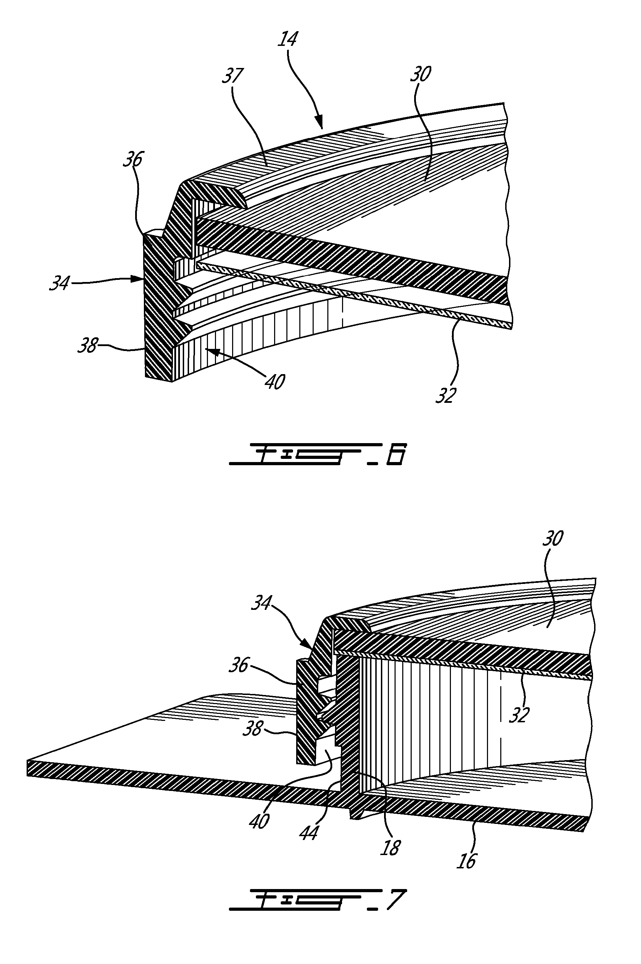

In case of a threaded screw-on ring 34, as illustrated herein, the inner surface 40 of the side wall 36 of the ring 34 comprises threads and an exterior surface 42 of the side wall 18 of the body 12 comprises external threads adjacent the open top 20 of the body 12 for mating with the threads on the ring 34, as best seen in Figure 7.

The outer surface 38 of the side wall 34 of the ring 32 may comprise dimples or ribs 50 as shown in Figures 1-5, 8 and 9, for a better grip by a user.

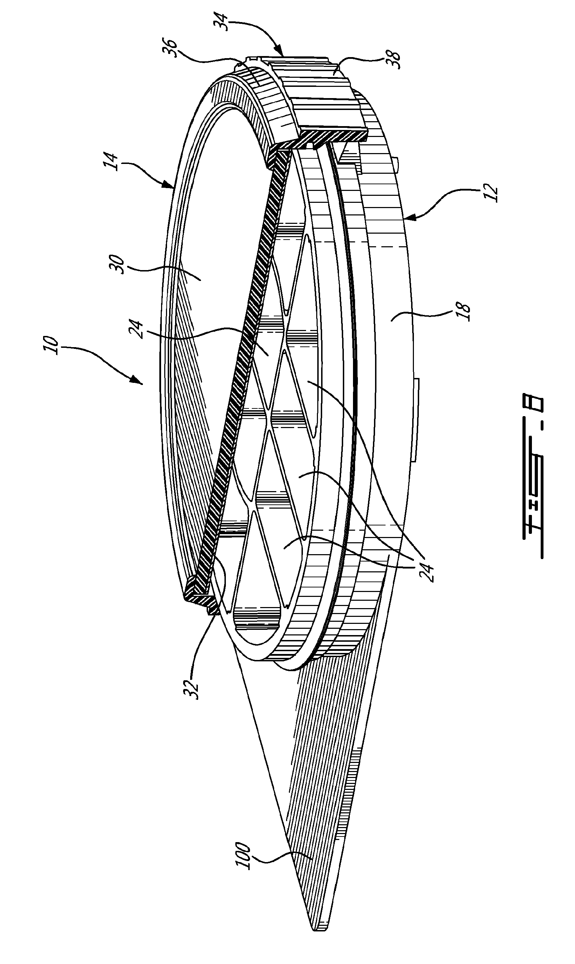

The ring 34 is used to cover the body 12 and hold the lid 30 in place over the open top 20 of the body 12. The ring 34, once properly screwed over the upper edge of the side wall 18 of the body 12, holds the lid 30 in place, which in turn holds the gasket 32 in place in contact with the upper edges of the walls 24 of each compartment 22a, 22b... (see Figure 8).

As best seen in Figure 6, the inner lid 30 is adapted to position itself under an edge 37 of the ring 34, above the threaded section of the inner surface 40 of the side wall 36 of the ring 34, in such a way to prevent the gasket 32, which is attached to the lid 30, from twisting as the ring 34 is secured to the upper edge of the side wall 18 of the body 12. Upon tightening the ring 34 by screwing or removing the ring 34 by unscrewing, only the ring 34 moves, the lid 30 and gasket 32 remaining in place over the compartments.

The gasket 32 may be attached to the lid 30 by welding or gluing or otherwise secured thereto.

Alternatively, the gasket 32 could be replaced by overmoulding a resilient plastic material over the top surface of the side wall 18 of the body 12 and over the top surface of the walls 24 of each compartment 22a, 22b... In this case, the lid 30 is held in place over the overmolded plastic material by tightening the ring 34.

The combination of the lid 30, gasket 32 and ring 34, once the ring 34 is properly secured over the upper edge of the side walls 16 of the body 12, forms a waterproof seal over the top of the body 12 and each compartment 22a, b.

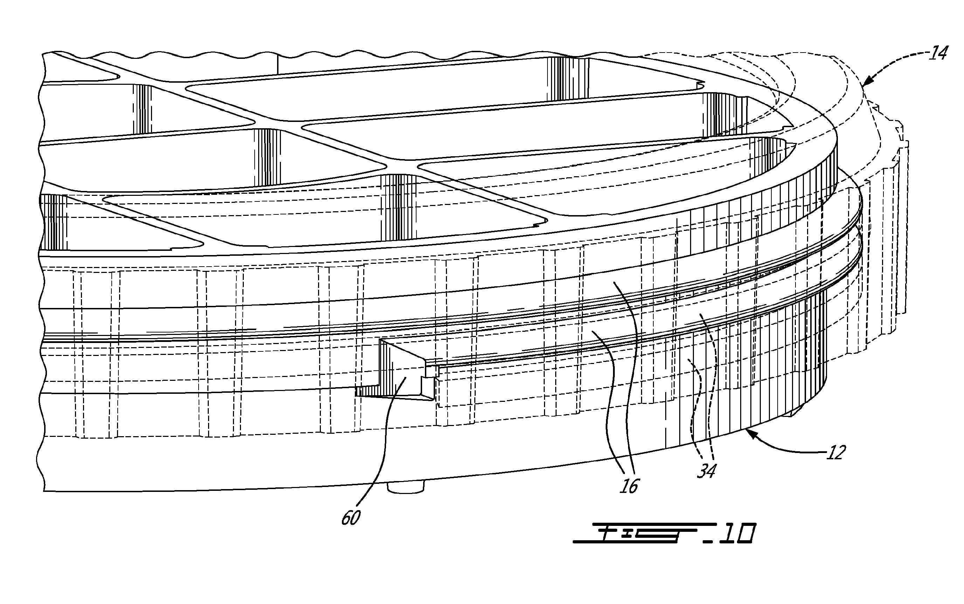

The side walls 16 of the body 12 may be provided with a stopper 60, which indicates that the user has reached the adequate tightness of the ring 34 about the upper edges of the side wall 16 of the body 12, and that the user cannot force beyond, thereby preventing damage to the ring 34 itself and/or the body 12 and lid 30 and its attached gasket 32 (see Figure 10). Moreover, this stopper 60 acts as a reminder to the user to properly tighten the ring 34, i.e. to tighten the ring 34 until abutment with the stopper 60 in case of a threaded screw-on ring 34 as illustrated herein, thereby preventing loose closing of the sampling dish 10, and risks of liquid and /or samples leakage.

The ring 34 could be a pop-on type, frictional fit, snap fit ... or provided with another interlocking structural engagement as known in the art.

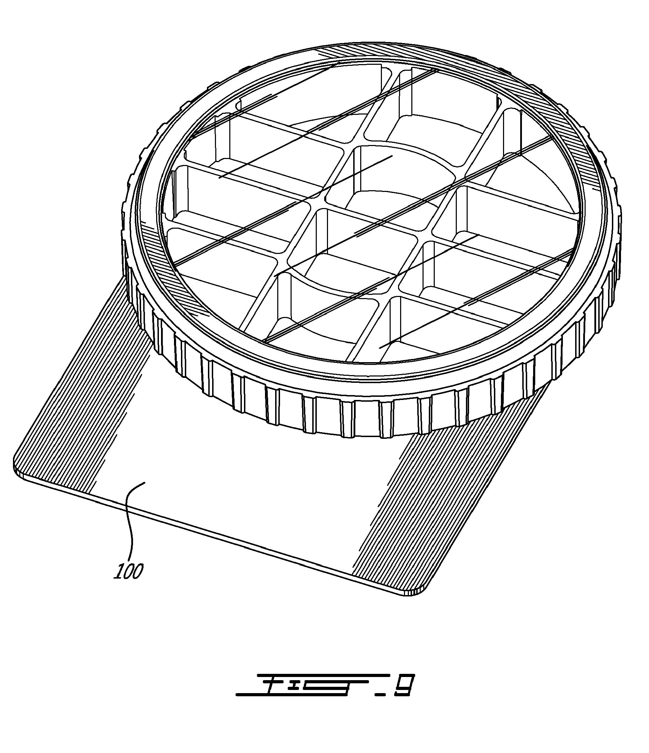

The body 12 and closure element 14 are typically made of transparent materials so as to allow internal viewing (see Figure 9), which do not interfere with the biological sample and is chemically inert when exposed to tissue preservatives, such as formalin for example.

The ring 34, as well as the body 12 and the lid 30, can be made in glass or any semi-rigid or rigid polymeric material such as, for example, polypropylene, polyethylene, polycarbonate, polystyrene, polyurethane etc.

The lid 30 and gaskets 34 are typically made in clear or translucent materials so that the contents of the compartments 22a, b ... can be viewed there through, as shown in Figures 8 and 9. The gasket 34 may be made in silicone, neoprene or polyethylene low density for example, for high mechanical resistance and resistance to organic solvents such as xylene, picric acid, alcohol, formalin. The gasket 34 may be glued to the lid 30. In case the cavity 22 in the body 12 only comprises one compartment, the gasket 34 may be provided only on the perimeter of the lid 30 for contact with the upper edges of the side wall 16 of the body 12 in the closed position of the closure element over the body 12.

Hermeticity of the sampling dish 10 as a whole, and tightness of each compartment 22a, b ..., is allowed by the present closure element arrangement, and allows a proper handling of biological sample, i.e. maintenance of the required amount of fixative agent and complete immersion of the sample if necessary for example,, which may be essential for accurate nuclei and RNA quantitative and qualitative evaluation for example.

Even if the sampling dish 10 happens to be handled or placed for a certain length of time in a position other than a, with the bottom wall of the base in a generally horizontal position, it is found that there is no circulation of fluid between the different compartments 22a, b..., regardless of the orientation of the sampling dish 10.

The present sampling dish is procedure-specific, and the compartments can be varied in size and number according to the samples typically collected from a particular site. For example, the present dish may be adapted for collecting, storing and transporting histological samples taken from the prostate, regions of the gastrointestinal tract, the cervix and the uterus, and other sites in humans and animals.

Typically, the size of each compartment is fairly small, so that locating a sample therein and recovering it therefrom is easy. Moreover, the amount of fixative agent needed to fill each compartment is thus reduced. The size of each compartment may be selected to provide the necessary volume for its target sample and fixative. Each compartment defines an individual watertight containment space when the closure element is secured on top of the base, as will be described hereinbelow, allowing that each biological sample remains uncontaminated by the other samples in the sampling dish.

Each compartment being differentiated by its label, the clinical personal is able to quickly identify an individual compartment. There is no need for further labelling, which in turn minimises the risk of mismatching.

As a result, the present one piece sampling dish provides the physician with an increased sampling flexibility and the closure element ensures hermeticity of the dish as a whole, and tightness of each compartment.

The present sampling dish can be prefilled with the reagent and shipped to the consumer or it can be shipped without a reagent therein and be filled with a suitable reagent at the time of use.

In use, the operator removes the lid and its attached gasket by removing the ring, to expose the open compartments. With an adequate fixative filling each compartment, the operator deposits the samples, each at a time, in the corresponding pre-labelled compartment. Then, the operator places the lid and its attached gasket on top of the compartments, and secures it into place by tightening the ring about the upper edges of the side wall of the body. The sampling dish can then be sent for analysis. At the location of analysis, the sampling dish is opened by releasing the ring to allow removing the lid and its attached gasket, and each sample can be picked up from its compartment for analysis.

The fixative may be liquid, or a gel or viscous or even semi solid material.

Although the present invention has been described hereinabove by way of embodiments thereof, it may be modified, without departing from the nature and teachings of the subject invention as defined in the appended claims.

## Claims

1. A sampling dish, comprising:
a body defining a cavity, said body comprising a bottom wall, an open top end and side walls extending from said bottom wall to said open top end;
a plate, and
a ring, said ring being movable into a mating position with edges of the side walls of said body to secure said plate in place over the open top of the body, thereby ensuring hermeticity of the dish in a closed position, and said ring being removable from said mating position to allow removal of said plate from said open top of the body for access to said cavity of the dish in an open position.

2. The sampling dish of claim 1, wherein said plate comprises a lid and a gasket attached to a surface of said lid facing said cavity.

3. The sampling dish of claim 1, wherein said cavity is divided into separate, adjacent compartments, and said ring secures said plate over the open top of the body, in contact with upper edges of walls of each of the compartments by mating with the edges of the side walls of said body thereby ensuring hermeticity of each compartment in the closed position of the dish.

4. The sampling dish of claim 1, wherein said ring is one of a threaded screw-on ring, a pop-on ring, a frictional fit ring and a snap fit ring.

5. The sampling dish of claim 1, wherein an inner surface of a side wall of said ring comprises threads and an exterior surface of the side walls of the body comprises external threads adjacent the open top of the body for mating with the threads on the side wall of the ring.

6. The sampling dish of claim 1, wherein said body and said plate are made in clear or translucent materials.

7. The sampling dish of claim 1, wherein said body is made in a biocompatible material chemically inert to tissue preservatives and selected in the group comprising glass, semi-rigid polymeric materials and rigid polymeric materials.

8. The sampling dish of claim 1, wherein said body is made in one of polypropylene, polyethylene, polycarbonate, polystyrene and polyurethane.

9. The sampling dish of claim 2, wherein said gasket is made in one of silicone, neoprene and polyethylene low density and said lid is made in one of polypropylene, polyethylene, polycarbonate, polystyrene and polyurethane.
